Europäisches Patentamt

European Patent Office

Office européen des brevets

(11) Publication number: **0 158 198**
**A1**

(12) **EUROPEAN PATENT APPLICATION**

(21) Application number: 85103450.4

(22) Date of filing: 23.03.85

(51) Int. Cl.⁴: **C 12 N 15/00**
C 12 P 19/34, C 12 P 21/02
C 07 K 7/10, C 07 K 15/26
//C12R1:185

(30) Priority. 29.03.84 PC T/JP84/00149
31.01.85 JP 18348/85

(43) Date of publication of application:
16.10.85 Bulletin 85/42

(84) Designated Contracting States:
CH DE FR GB LI

(71) Applicant. Takeda Chemical Industries, Ltd.
27, Doshomachi 2-chome Higashi-ku
Osaka-shi Osaka, 541(JP)

(72) Inventor: Senoo, Masaharu
D73-106, 18 Tuskumodai 5-chome
Suita-shi Osaka 565(JP)

(72) Inventor: Onda, Haruo
21-6, Aza-junmatsu Tada-in
Kawanishi-shi Hyogo 666-01(JP)

(72) Inventor: Igarashi, Koichi
C3-303, 3 Takenodai
Nagaokakyo-shi Kyoto 617(JP)

(74) Representative: von Kreisler, Alek, Dipl.-Chem. et al,
Deichmannhaus am Hauptbahnhof
D-5000 Köln 1(DE)

(54) DNA and use thereof.

(57) A polypeptide comprising amino acid sequence of human interferon and that of human interleukine-2-may be produced by making new DNA by the ligation of DNA containing the structural gene coding for the peptide of human interferon with DNA containing the structural gene coding for human interleukine-2, transforming the host cells with the DNA, and cultivating the transformant.

DNA and Use thereof

This invention relates to DNA that produces polypeptides which can be used as reagent in the purification of human interleukin-2 antibody, which DNA is formed by the ligation of DNA bearing the structural gene coding for the peptide of human immune interferon (hereafter, human IFN-$\gamma$) and DNA coding for the peptide of human interleukin-2 (hereafter, human IL-2), and to the uses thereof.

Interferons are proteins produced by the cells of higher animals when stimulated by viruses, nucleic acids, and other agents, these have antiviral, antitumor, and other activities. Interferons are currently classified into three types according to their characteristic properties; namely, $\alpha$, $\beta$ and $\gamma$ types. $\gamma$-type interferon is called immune interferon because it is produced from immunologically competent cells under those circumstances under which lymphocyte transformation (the conversion to blast cells) or lymphokine production take place. $\gamma$-type interferon is said to have higher anti-cell-proliferation and antitumor activities than $\alpha$-type and $\beta$-type interferon, and therefore so much more is expected from the point of clinical applications.

However, due to various limitations, such as the requirement for fresh lymphocytes in the production of $\gamma$-type interferon, an efficient production system using natural sources has not been established yet. It is possible at present to obtain large amounts of human immune interferon by culturing the transformant obtained by transformation with a plasmid bearing the structural gene coding for the peptide of human IFN-$\gamma$. [Nature 295, 503 (1982); Nucleic Acids Research 10, 2487 (1982); Nucleic Acids Research 10, 3605 (1982); Nucleic Acids Research Symposium Series No.11, 29 (1982); Abstract of 4th International Symposium on Genetics of Industrial Microorganisms Kyoto p.30 (1982)]

Meanwhile interleukin-2 [hereafter, IL-2; formerly called T cell growth factor (TCGF)]is a lymphokine produced by T cells upon stimulation with substances such as a lectin or alloantigen, for instance [Science 193, 1007 (1976); Immunological Review 51, 257 (1980)]. IL-2 enables T cells to grow in vitro for an extended period of time, while maintaining their functions. Furthermore, IL-2 is reported promotes the mitogen reaction of thymus cells (costimulator), to restore the production by spleen cells of antibodies against T cell-dependent antigens in nude mice (T cell-replacing factor) and to promote the differentiation and proliferation of killer cells (killer helper factor) [The Journal of Immunology 123, 2928 (1979); Immunological Review 51, 257 (1980)].

However, it is difficult to obtain large amounts of human IL-2 from natural sources. It is now possible to mass-produce human IL-2 by culturing the transformant obtained with a plasmid containing the integrated structural gene of human IL-2 using gene manipulation techniques [Nature 302, 305 (1983); Biochemical and Biophysical Research Communications 109, No.2, P.363 (1982); Nucleic Acids
Research, 11, 4307 (1983)].

As mentioned above the expression of the structural
genes coding individually for human IFN-γ and human IL-2 have
been studied independently.

The object of this invention is the preparation of a
novel conjugated dissimilar proteins that have two different
immunological or biological activities.

We have made a hybrid protein consisting of human
IFN-γ and human IL-2 by employing the techniques of genetic
engineering and have found this protein to have the antiviral
activity and antigenicity of human IFN-γ as well as the T
cell growth activity and antigenicity of human IL-2, we
perfected the present invention based on these findings.

The present invention provides; (1) a DNA made by
ligating a DNA containing a structural gene coding for a
peptide of human IFN-γ and a DNA containing a structural gene
coding for a peptide of human IL-2 to code the peptide of
human IFN-γ and the peptide of human IL-2 in a single reading
frame, (2) a plasmid carrying a DNA made by ligating a DNA
containing a structural gene coding for a peptide of human
IFN-γ and a DNA containing a structural gene coding for a
peptide of human IL-2 to code the peptide of human IFN-γ and
the peptide of human IL-2 in a single reading frame, (3) a
transformant transformed by a plasmid carrying a DNA made by
ligating a DNA containing a structural gene coding for a
peptide of human IFN-γ and a DNA containing a structural gene

- 4 -

0158198

coding for a peptide of human IL-2 to code the peptide of human IFN-$\gamma$ and the peptide of human IL-2 in a single reading frame, (4) a polypeptide comprising a peptide of human IFN-$\gamma$ and a peptide of human IL-2, and (5) a method for producing the polypeptide mentioned above (4) which comprises cultivating a transformant transformed by a plasmid carrying a DNA made by ligating a DNA containing a structural gene coding for a peptide of human IFN-$\gamma$ and a DNA containing a structural gene coding for a peptide of human IL-2 to code the peptide of human IFN-$\gamma$ and the peptide of human IL-2 in a single reading frame in a medium, producing and accumlating the polypeptide comprising of the peptide of human IFN-$\gamma$ and the peptide of human IL-2 in the culture broth, and isolating the polypeptide from the broth.

In the present specification, the polypeptide comprising  the peptide of human IFN-$\gamma$ and the peptide of human IL-2 is called interleuron$\gamma$ 2, or simply ILR-$\gamma$ 2.

A peptide of human IFN-$\gamma$ used in the present invention includes any of peptide containing the characteristic activity of human IFN-$\gamma$ . For example, human IFN-$\gamma$ is originally consists of 146 amino acids, yet the peptide of human IFN-$\gamma$ used in the present invention may lack the amino terminal and carbonyl terminal residues or have additional amino acids at the amino and carbonyl terminal, but must retain the characteristic activity of human IFN-$\gamma$ .

In the present invention, a peptide which lacking the 11 amino acids at the carboxy terminal end is preferred.

0158198

One example of the structural gene coding for the peptide of human IFN-$\gamma$ used in the present invention is the gene coding for the peptide with a molecular weight of 17000$\pm$1000 and antiviral and antitumor activities that was described in Nature 295, 503 (1982), Nucleic Acids Research 10, 2487 (1982), Nucleic Acids Research 10, 3605 (1982), Japanese Patent Provisional Publication Nos.58-90514(EPC Provisional Publication No.77670) and 58-189197(EPC Provisional Publication No.89676), Japanese Patent Application Nos 58-176090[Provisional Publication No.59-186995 (EPC Provisional Publication No.110044)],and 58-45723 [Provisional Publication No. 59-169494 (EPC Provisional Publication No.126230)]

Japanese Patent Provisional Publication No. 58-189197 (EPC Provisional Publication No.89676) mentions DNA containing the base sequence:

```
        1
(5')TGT TAC TGC CAG GAC CCA TAT GTA AAA GAA GCA GAA AAC CTT AAG
    AAA TAT TTT AAT GCA GGT CAT TCA GAT GTA GCG GAT AAT GGA ACT CTT
    TTC TTA GGC ATT TTG AAG AAT TGG AAA GAG GAG AGT GAC AGA AAA ATA
    ATG CAG AGC CAA ATT GTC TCC TTT TAC TTC AAA CTT TTT AAA AAC TTT
    AAA GAT GAC CAG AGC ATC CAA AAG AGT GTG GAG ACC ATC AAG GAA GAC
    ATG AAT GTC AAG TTT TTC AAT AGC AAC AAA AAG AAA CGA GAT GAC TTC
    GAA AAG CTG ACT AAT TAT TCG GTA ACT GAC TTG AAT GTC CAA CGC AAA
    GCA ATA CAT GAA CTC ATC CAA GTG ATG GCT GAA CTG TCG CCA GCA GCT
                                                        140
    AAA ACA GGG AAG CGA AAA AGG AGT CAG ATG CTG TTT CGA GGT CGA AGA
        146
    GCA TCC CAG-X (3')                    (I)
```

[wherein X is TAA, TGA, or TAG]

This codes for the polypeptide:

(N) Cys Tyr Cys Gln Asp Pro Tyr Val Lys Glu Ala Glu Asn Leu Lys

Lys Tyr Phe Asn Ala Gly His Ser Asp Val Ala Asp Asn Gly Thr Leu

Phe Leu Gly Ile Leu Lys Asn Trp Lys Glu Glu Ser Asp Arg Lys Ile

Met Gln Ser Gln Ile Val Ser Phe Tyr Phe Lys Leu Phe Lys Asn Phe

Lys Asp Asp Gln Ser Ile Gln Lys Ser Val Glu Thr Ile Lys Glu Asp

Met Asn Val Lys Phe Phe Asn Ser Asn Lys Lys Lys Arg Asp Asp Phe

Glu Lys Leu Thr Asn Tyr Ser Val Thr Asp Leu Asn Val Gln Arg Lys

Ala Ile His Glu Leu Ile Gln Val Met Ala Glu Leu Ser Pro Ala Ala

Lys Thr Gly Lys Arg Lys Arg Ser Gln Met Leu Phe Arg Gly Arg Arg

Ala Ser Gln (C)        (II)

or polypeptides with equivalent immunological or biological
activities.

DNA (I) above may have ATG (III) at the 5' terminal.
When DNA (I) has ATG (III) at the 5' terminal, the DNA codes
for the polypeptide consisting of polypeptide (II) to which
is added Met at the N-terminal end or a polypeptide with
equivalent activity. [See Japanese Patent Application No.58-
45723(Provisional Publication 59-169494, EPC Provisional
Publication No.126230]

The DNA chemically synthesized by Tanaka et al. and
containing the base sequence:

```
1                                        10
TGC TAC TGC CAG GAC CCA TAC GTG AAG GAA GCT GAA AAC CTG AAG A
              20                                  30
TAC TTC AAC GCT GGT CAT TCT GAC GTT GCT GAC AAC GGT ACT CTG T
                            40
CTG GGT ATC CTG AAA AAC TGG AAA GAA GAA TCT GAC CGT AAA ATC A
    50                                        60
CAG TCT CAG ATC GTT TCT TTC TAC TTC AAG CTG TTC AAA AAC TTC A
```

```
                    70                                          80
GAC GAC CAG TCT ATC CAG AAA TCT GTT GAA ACT ATC AAG GAA GAC ATG
                                     90
AAC GTT AAG TTC TTC AAC TCT AAC AAG AAA AAG CGT GAC GAC TTC GAA
        100                                           110
AAG CTT ACT AAC TAC TCT GTT ACT GAC CTT AAT GTA CAG CGT AAA GCT
                            120
ATC CAT GAA CTG ATC CAG GTT ATG GCT GAA CTG TCC CCG GCT GCT AAA
    130                                     140
ACT GGT AAG CGT AAA AGA TCT CAG ATG CTG TTC CGT GGT CGT CGT GCT
    146
TCT CAG       (IV)
```

Also serves as an example of a structural gene coding for an IFN- peptide.

[Nucleic Acids Research Symposium Series No.11, 29-32(1982)].

Another example is the DNA described in Japanese Patent Provisional Publication No.58-201995(EPC Provisional Publication No.95350), and No.59-51792 (EPC Provisional Publication No.88540).

The peptide of human IL-2 refered to in the present invention may be any a peptide having the activity of human IL-2; this may be a smaller peptide or a bigger peptide, provided it has the activity of human IL-2.

Examples of the structural genes coding for such a human IL-2 peptide, include the DNA segments described in Japanese Patent Application No.58-225079 [application date, Nov. 28, 1983,(EPC Patent Application No.84 308153.0)], Japanese Patent Application No. 58-235638 (application date, Dec. 13, 1983), Nature 302, 305(1983), Biochemical and Biophysical Research Communications 109, No. 2,363 (1982), and Nucleic Acids Research 11, 4307 (1983).

Codons 1-133(V) in the following base sequence:

5' GGGGGGGGGGGGGGGGGGGGATCACTCTCTTTAATCACTACTCACAGTAACCTCAA
      S1

CTCCTGCCACA ATG TAC AGG ATG CAA CTC CTG TCT TGC ATT GCA CTA AGT
                S20 1

CTT GCA CTT GTC ACA AAC AGT GCA CCT ACT TCA AGT TCT ACA AAG AAA
                                  20

ACA CAG CTA CAA CTG GAG CAT TTA CTG CTG GAT TTA CAG ATG ATT TTG
                                        40

AAT GGA ATT AAT AAT TAC AAG AAT CCC AAA CTC ACC AGG ATG CTC ACA

TTT AAG TTT TAC ATG CCC AAG AAG GCC ACA GAA CTG AAA CAT CTT CAG
      60

TGT CTA GAA GAA GAA CTC AAA CCT CTG GAG GAA GTG CTA AAT TTA GCT
                              80

CAA AGC AAA AAC TTT CAC TTA AGA CCC AGG GAC TTA ATC AGC AAT ATC
                                    100

AAC GTA ATA GTT CTG GAA CTA AAG GGA TCT GAA ACA ACA TTC ATG TGT
                                    120

GAA TAT GCT GAT GAG ACA GCA ACC ATT GTA GAA TTT CTG AAC AGA TGG
                                133

ATT ACC TTT TGT CAA AGC ATC ATC TCA ACA CTG ACT TGA TAATTAAGTGC

TTCCCACTTAAAACATATCAGGCCTTCTATTTATTTAAATATTTAAATTTTACCCCCCCCCCC

CCCC 3'

may be cited as a DNA sequence that codes for the human IL-2 peptide described in Japanese Patent Application No.

58-225079 above.

      This DNA segment (V) may have ATG or the signal codon

represented codons $S_1$-$S_{20}$ in the above formula at the 5' terminal and may have TAA, TGA, or TAG, and especially TGA, at the 3' terminal.

DNA(V) above codes for the following peptide:
  1
X - Ala Pro Thr Ser Ser Ser Thr Lys Lys Thr Gln Leu Gln Leu Glu
                20

His Leu Leu Leu Asp Leu Gln Met Ile Leu Asn Gly Ile Asn Asn Tyr
                            40

Lys Asn Pro Lys Leu Thr Arg Met Leu Thr Phe Lys Phe Tyr Met Pro
                                60

Lys Lys Ala Thr Glu Leu Lys His Leu Gln Cys Leu Glu Glu Glu Leu

Lys Pro Leu Glu Glu Val Leu Asn Leu Ala Gln Ser Lys Asn Phe His
80
Leu Arg Pro Arg Asp Leu Ile Ser Asn Ile Asn Val Ile Val Leu Glu

                          100
Leu Lys Gly Ser Glu Thr Thr Phe Met Cys Glu Tyr Ala Asp Glu Thr
                                                120
Ala Thr Ile Val Glu Phe Leu Asn Arg Trp Ile Thr Phe Cys Gln Ser
                                 133
Ile Ile Ser Thr Leu Thr.

(wherein X is Met or hydrogen)

The DNA coding for the human IL-2 peptide described in above-cited Japanese Patent Application No.58-235638 has the following base sequence:

(5') X-AGT TCT ACA AAG AAA ACA CAG CTA CAA CTG GAG CAT TTA CTG CTG GAT TTA CAG ATG ATT TTG AAT GGA ATT AAT AAT TAC AAG AAT CCC AAA CTC ACC AGG ATG CTC ACA TTT AAG TTT TAC ATG CCC AAG AAG GCC ACA GAA CTG AAA CAT CTT CAG TGT CTA GAA GAA GAA CTC AAA CCT CTG GAG GAA GTG CTA AAT TTA GCT CAA AGC AAA AAC TTT CAC TTA AGA CCC AGG GAC TTA ATC AGC AAT ATC AAC GTA ATA GTT CTG GAA CTA AAG GGA TCT GAA ACA ACA TTC ATG TGT GAA TAT GCT GAT GAG ACA GCA ACC ATT GTA GAA TTT CTG AAC AGA TGG ATT ACC TTT TGT CAA AGC ATC ATC TCA ACA CTG ACT-Y(3')   (VI)

(wherein X is hydrogen or a codon other than AGC, AGT, TCA, TCC, TCG, TCT, TAA, TAG, and TGA, and Y is a codon or a hydroxyl group).

DNA(VI) codes for the peptide having the following sequence:

Z-Ser Ser Thr Lys Lys Thr Gln Leu Gln Leu Glu His Leu Leu Leu Asp Leu Gln Met Ile Leu Asn Gly Ile Asn Asn Tyr Lys Asn Pro Lys Leu Thr Arg Met Leu Thr Phe Lys Phe Tyr Met Pro Lys Lys Ala Thr Glu Leu Lys His Leu Gln Cys Leu Glu Glu Glu Leu Lys Pro Leu Glu Glu Val Leu Asn Leu Ala Gln Ser Lys Asn Phe His Leu Arg Pro Arg Asp Leu Ile Ser Asn Ile Asn Val Ile Val Leu Glu Leu Lys Gly Ser Glu Thr Thr Phe Met Cys Glu Tyr Ala Asp Glu Thr Ala Thr Ile Val

Glu Phe Leu Asn Arg Trp Ile Thr Phe Cys Gln Ser Ile Ile Ser Thr Leu Thr (VII) (wherein Z is hydrogen or an amino acid residue other than Ser).

The codon represented by X in DNA segment(VI) may be any codon that codes for an amino acid forming a part of the polypeptide(VII) having substancially the same activity as human IL-2 (providing X is neither a codon that specifies Ser nor a termination codon) and it may have at least one codon that specifies an amino acid at the 5' terminal. ATG is most preferable as X.

The codon represented by Y may be either a termination codon or a codon that specifies an amino acid forming a part of the polypeptide(VII) having substantially the same activity as human IL-2, and it may have at least one codon that specifies an amino acid at the 3' end. When the codon represented by Y is a codon other than a termination codon (including cases where it has at least one codon that specifies an amino acid at the 3' end), the sequence shall have a termination codon at the 3' end. TAA, TAG or TGA are the preferred choices as the codon represented by Y; of these, TGA is especially preferable.

As for polypeptide (VII), any amino acid residue, such which forms a part of the polypeptide having substantially the activity of human IL-2 may be used as the amino acid other than Ser represented by Z. Examples include those shown in Table 1 (excluding Ser). Moreover, the polypeptide may have an amino acid residue shown in Table 1, or a peptide made up of several such amino acid residues at the N-terminal end. Met or hydrogen are most preferable as Z.

The reading frames of a DNA segment containing the structural gene coding for the human IFN-$\gamma$ peptide and a DNA segment containing the structural gene coding for the human IL-2 peptide are fused into one frame by ligating.

Ligation may be carried out by a known method, such as ligaton of the 3'-OH terminal end of one DNA segment and the 5'-P terminal end of the other DNA segment with T4 DNA ligase in the presence of ATP.

A linker should preferably be used between the two structural genes.

The linker may be, for example, EcoRI linker p(CCGGAATTCCGG) available commercially from New England Biolabs (U.S.A.), EcoRI linker p(CCGGAATTCCGG), EcoRI linker p(TGCCATGAATTCATGGCA) and so forth.

The linker may be joined as follows; an E. coli polymerase I (large fragment) or Sl nuclease is used to make a flush end at the terminal of one gene, and the resulting flush end is joined with the end of the linker by T4 DNA ligase. To avoid the ligation of linkers themselves, digestion by an appropriate restriction enzyme is required. When an EcoRI linker is used, EcoRI may be used as the restriction enzyme. By using the sticky ends thus obtained, one may obtain a single DNA segment between consisting of the two genes connected by a linker ligated to the genes with T4 DNA ligase.

A promoter should preferably be connected upstream from the DNA formed by ligating the two genes.

The two structural genes connected downstream from the promoter may be in the following order, going downstream from the promotor: the structural gene for human IFN-$\gamma$ followed by the structural gene for human IL-2, or the structural gene for human IL-2 followed by the structural gene for human IFN-$\gamma$. Although the order of ligation of the two structural genes is not of crucial importance, the promoter must be connected upstream from the genes.

A tryptophan(trp) promoter, recA promoter, or lactose promoter may be used, as the promoter. Of these, a trp promoter is especially preferable.

The promoter may be ligated by connecting the initiation codon ATG to the 5' end of the gene to be expressed by using as appropriate linker, and ligating the linker to the 3' end of the promoter. T4 DNA ligase is generally used as the enzyme in this process. The same method is used as that described above. A recognition site for the restriction enzyme shall preferably be provided downstream from the promoter.

A plasmid may be used as the vector that expresses the DNA formed by the ligation of these two genes in the host organism, in which case the new DNA is inserted into the plasmid.

The plasmid pBR322 from Col. EI [Gene 2, 95 (1977)] is most frequently used as the above vector but any other plasmid that can efficiently express this DNA may be used. Additional examples, include the plasmids ptrp 781 and ptrp 771 into which a tryptophan promoter has been inserted.

To insert the DNA thus obtained into the plasmid the plasmid DNA must first be made linear by treatment with an appropriate restriction enzyme, followed by ligation of the plasmid DNA with the new DNA obtained above using T4 DNA ligase.

A transformant is then obtained by transforming the host organism with the plasmid containing the inserted DNA prepared by ligation of the two genes.

The host may be, for example, an organism of the genus Escherichia, Bucillus subtilis, or a yeast, an organism of the genus Escherichia being especially preferable.

The above Escherichia organism may be, for example, a strain of E. coli DH1 [Nature 217, 1110-1114 (1968)], Journ. of Mol. Biology 166, 557-580 (1983).
The host cell may be transformed by the plasmid using the calcium method, protoplast method, or some other method; use of the Ca method is especially preferable.

One example of a transformant obtained by the above method was Escherichia coli DH1/pIFL9906, prepared in Example 1. This microorganism was deposited at the Institute for Fermentation, Osaka(IFO), Japan (17-85, Jusohonmachi 2-chome, Yodogawa-ku, Osaka, 532, Japan) under accession number IFO 14331 on March 27, 1984. This microorganism was deposited at the Fermentation Research Institute, Agency of Industrial Science and Technology, Ministry of International Trade and Industry(FRI), Japan(1-3, Yatabe-machi higashi 1-chome, Tsukuba-gun, Ibaragi Prefecture, Japan) under accession number FERM P-7567 on March 29, 1984, and this deposit was converted to a deposit under the Budapest Treaty, and the strain has been stored at the FRI under accession number FERM BP-711.

A liquid medium is appropriate, as the medium for cultivating the transformant thus obtained; this must include a carbon source, a nitrogen source, minerals, and other nutrients necessary for growth of the transformant. The carbon source may be, for example, glucose, dextrin, water-soluble starch, or sucrose, while the nitrogen source, which may comprises organic or inorganic compounds, may be, for example, ammonium salts, nitrates, corn-steep liquor, peptone, casein, meat extract, soybean meal, or potato extract. The minerals may be, for instance, calcium chloride, sodium dihydrogenphosphate, or magnesium chloride.

Yeast extract, vitamins, and growth promoting factor may be added. Medium containing 0.1% calcium chloride dihydrate and 2% sodium dihydrogenphosphate dihydrate is especially useful for production of the desired product. The incubation conditions, such as temperature, pH, and incubation period, may be set as required to maximize the production and activity of the desired product. A temperature of about 20 to 40 °C, a pH ranging from weak acidity to weak alkaline (especially near neutrality), and an incubation time of about 6 to 10 hours are generally preferable.

The desired polypeptide is stored in the cultured organisms, incubation, and may be obtained by centrifugation or filtration of the organisms from the broth, followed by extraction of the proteins. The polypeptide may be efficiently extracted by any suitable technique, including ultrasound treatment, lysozyme treatment, or treatment with chemicals such as surfactants.

The polypeptide thus obtained may be purified by a known protein or peptide purification method such as ammonium sulfate precipitation, salting out, alcohol precipitation, cellulose column chromatography, or gel filtration.

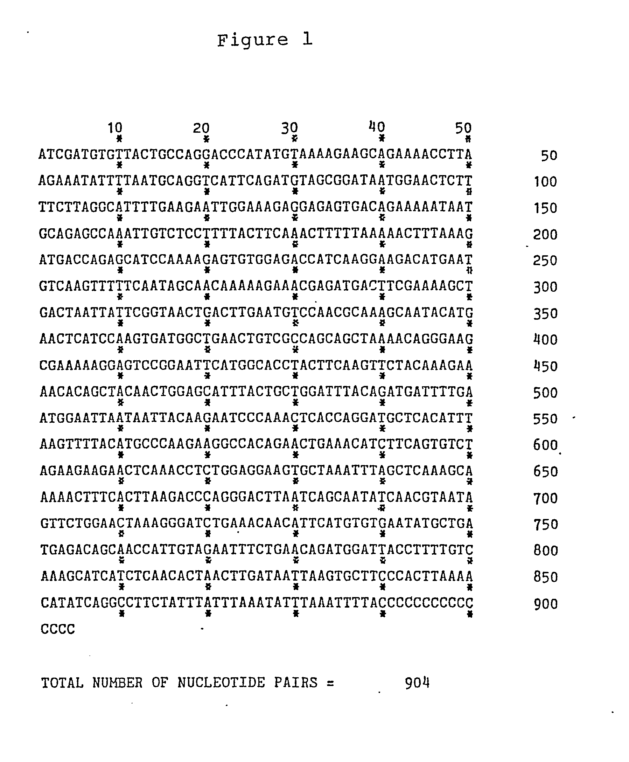

The DNA obtained in Example 1, described later, contains a polynucleotide having the nucleotide sequence shown in Fig. 1.

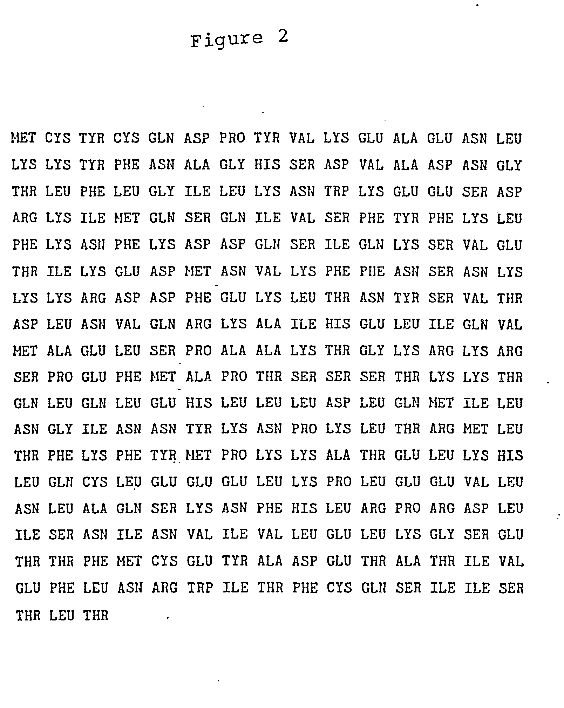

The polynucleotide consisting of nucleotide sequence 8-412 in Fig. 1 codes for the polypeptide represented as amino acid sequence 2-137 in Fig. 2, namely human IFN-$\gamma$.

And the polynucleotide represented as nucleotide sequence 425-823 in Fig. 1 codes for the polypeptide represented as amino acid sequence 142-274 in Fig. 2, namely human IL-2.

These polynucleotides may have an ATG codon for initiation of the reading frame at the 5' end for their direct expression. This codes for a peptide having Met at

the NH$_2$-terminal end.

These polynucleotides may have an adequate intercalary sequence to fuse the two independent reading frames for human IFN-γ and human IL-2 into one. In Fig. 1 the intercalary sequence is nucleotide sequence 413-424.

This codes for the polypeptide represented as amino acid sequence 138-141 in Fig. 2.

By using ILR-γ2 according to the present invention, human IL-2 can be refined effectively. And ILR-γ2 according to the present invention may be used as an anti-tumor agent, etc.

As mentioned above, the mRNAs coding for human IFN-γ and human IL-2 have already been identified independently, and that the expression of these genes attempted, also independently. However, this invention uses a new concept: the production of a new polypeptide having two or more different immunological or biological activities by the ligation of two or more different genes. Furthermore, polypeptides having two or more independent immunological or biological activities are rare in nature. Hence, this invention, because it provides a new type of protein, is a pioneering in the field of the protein engineering founded on genetic engineering.

Because ILR-γ2 with this amino acid sequence has the antigenicities of both human IFN-γ and human IL-2, it is useful as the medium or reagent for the series of human IL-2 purification processes comprising the purification of ILR-γ2 by anti-human IFN-γ antibody, the production and purification of antihuman IL-2 antibody with the purified ILR-γ 2, and the purification of human IL-2 with the purified anti-human IL-2 antibody. Here, ILR-γ2 plays a direct role only in purification of the anti-human IL-2 antibody, but as no other method such as this exists for the production and purification of unknown antibodies much is expected as a new technology for antibody purification.

ILR-γ2 has the immunological or biological activities of both human IFN-γ , which plays a part in antiviral action, and human IL-2 which has an ability to support T cell growth. Further ILR-γ2 of the present invention has natural killer cell activity.

The following method may be used to purify human IL-2 antibody.

First, the antibody column is made by binding anti-human IFN-γ antibody and agarose gel beads (Bio-Rad, Affigel 10). ILR-γ2 in the extract of E.coli DH1/pIFL9906 is then bounded specifically to this antibody column.

Next, rat ascitic fluid or rabbit serum immunized with human IL-2 is reacted with this IFN-γ antibody column containing adsorbed ILR-γ 2. A solution containing ILR-γ2 was then obtained by eluting the column with a suitable buffer solution. The anti-human IL-2 antibody was obtained by eluting this solution at a salt concentration at which a DEAE cellulose column (Whatman Co., Cellulose 52) adsorbs proteins other than antibodies.

The ILR-$\gamma$ 2 of the present invention has the both antiviral action of human IFN-$\gamma$ and the ability to support T-cell growth action of IL-2. Moreover, the ILR-$\gamma$2 of the present invention has natural killer cell activity, which enables it to be used in the prevention and treatment of virus-induced diseases, tumors, and immunodeficiency disorders in warm-blooded mammals (e.g., mice, rats, rabbits, dogs, cats, pigs, horses, sheep, cattle, and human).

The ILR-$\gamma$2 protein prepared according to the present invention is of high purity, and so has low toxicity and no antigenicity.

The ILR-$\gamma$2 of the present invention may be used as an antiviral agent, an antitumor agent, or an immunopotentiating agent by enteral or parenteral administration as an injection, capsule, or the like together with a carrier, excipient, diluent, or other known and pharmacologically allowed adjuvants.

The daily dosage when administered as above in warmblooded mammals should be from about 1 to 100 $\mu$g/kg, and preferably from about 1 to 10 $\mu$g/kg.

In the present specification and drawings, the nucleotides and amino acids, when indicated by abbreviations, are abbreviated according to the rules of the IUPAC-IUB Commission on Biochemical Nomenclature or the conventions currently used in the art. Examples are shown in Table 1. In cases where an optical isomer may exist, the amino acids indicated are in the L-form unless otherwise noted.

Table 1

| DNA: | Deoxyribonucleic acid |
|---|---|
| cDNA: | Complementary deoxyribonucleic acid |
| RNA: | Ribonucleic acid |
| mRNA: | Messenger ribonucleic acid |
| A: | Adenine |
| T: | Thymine |
| G: | Guanine |
| C: | Cytosine |
| U: | Uridine |
| dATP: | Deoxyadenosine triphosphate |
| dTTP: | Thymidine triphosphate |
| dGTP: | Deoxyguanosine triphosphate |
| dCTP: | Deoxycytidine triphosphate |
| ATP: | Adenosine triphosphate |
| EDTA: | Ethylenediaminetetraacetic acid |
| SDS: | Sodium dodecyl sulfate |
| Gly: | Glycine |
| Ala: | Alanine |
| Val: | Valine |
| Leu: | Leucine |
| Ile: | Isoleucine |
| Ser: | Serine |
| Thr: | Threonine |
| Cys: | Cysteine |
| Met: | Methionine |
| Glu: | Glutamic acid |

Table 1(continued)

Asp:    Aspartic acid

Lys:    Lysine

Arg:    Arginine

His:    Histidine

Phe:    Phenylalanine

Tyr:    Thyrosine

Trp:    Tryptophan

Pro:    Proline

Asn:    Asparagine

Gln:    Glutamine

bp:     base pair

Brief Description of the Drawings

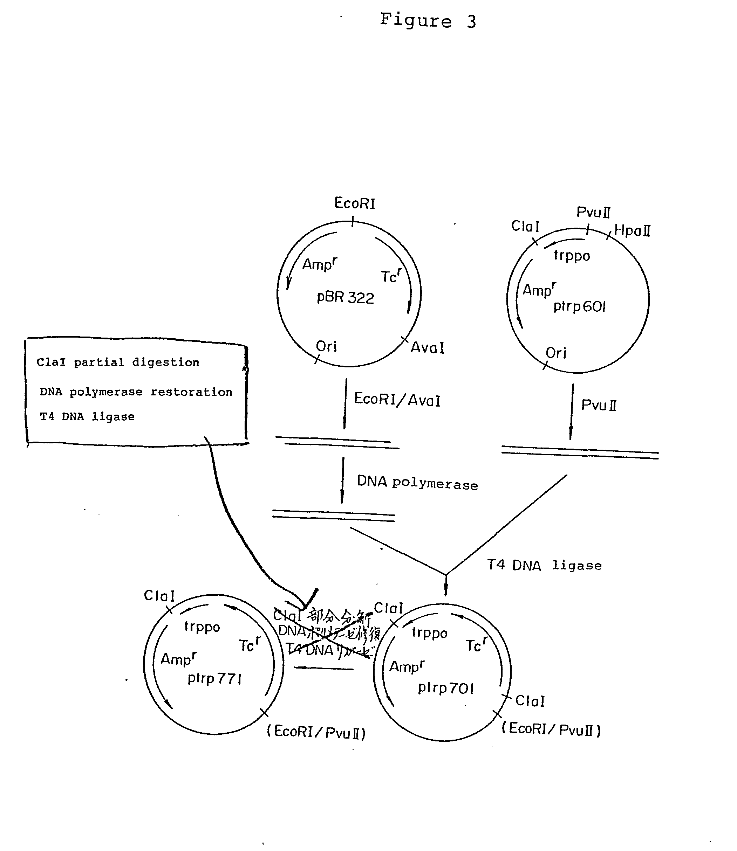

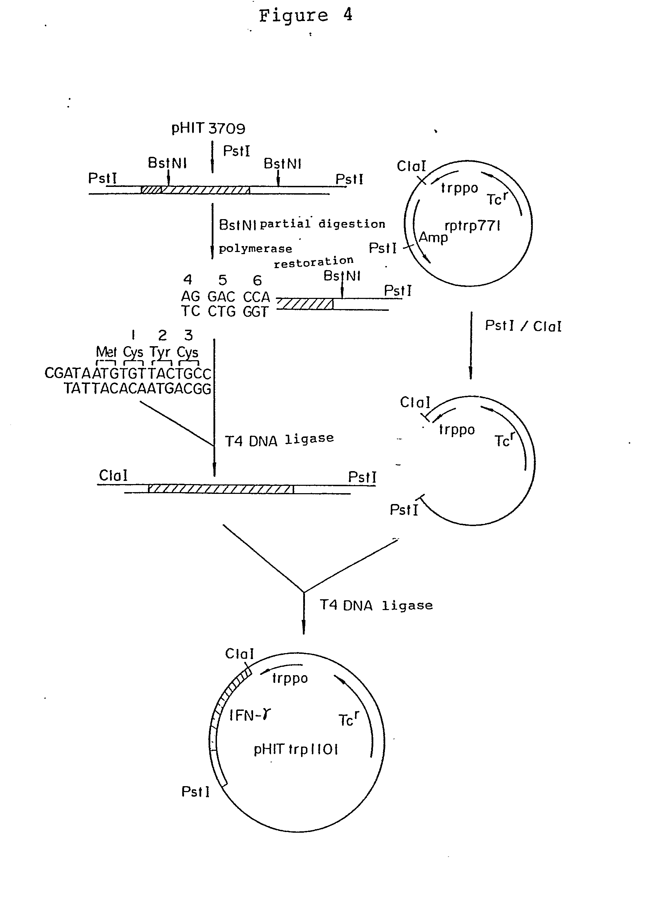

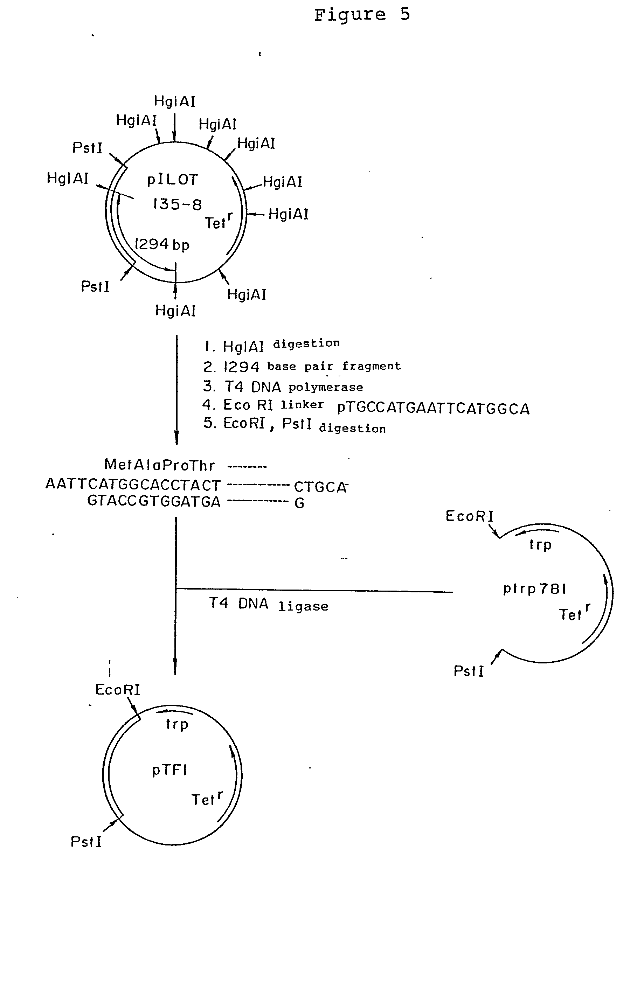

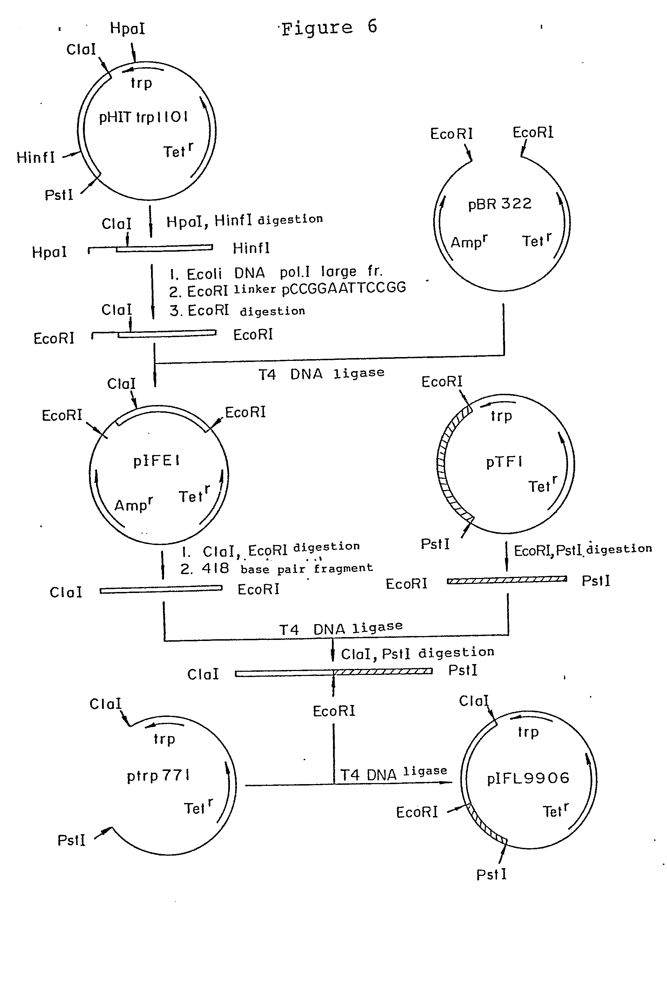

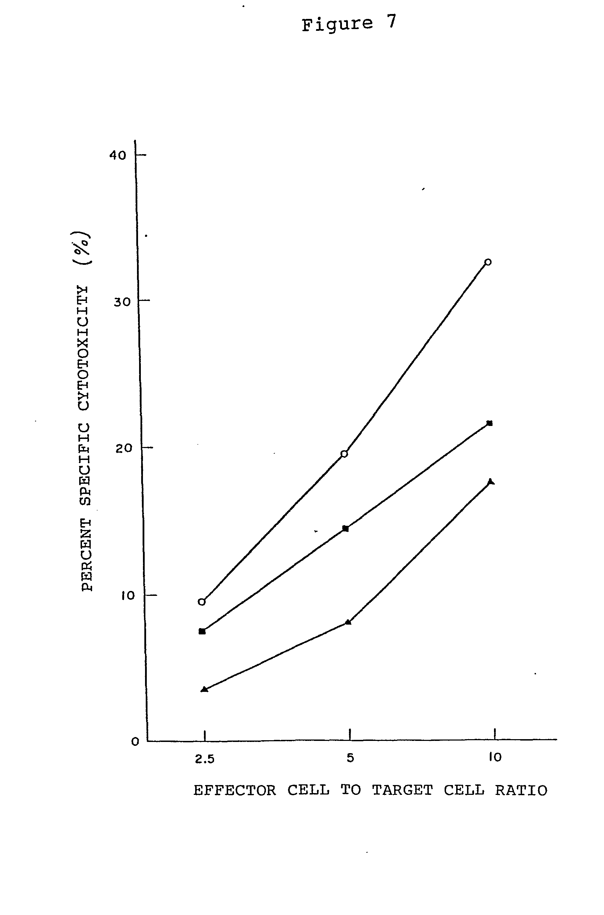

        Fig. 1 shows the nucleotide sequence prepared by ligating a DNA containing the structural gene coding for human IFN-γ peptide and a DNA containing the structural gene coding for human IL-2 peptide. Fig. 2 shows the amino acid sequence coded for by the nucleotide sequence in Fig. 1. Fig. 3 shows the construction in Reference Example 1(1) scheme for the plasmid ptrp 771. Fig. 4 shows the construction scheme in Reference Example 1 (2) for the plasmid pHITtrp 1101 ( ///// indicates the portion coding for human IFN-γ peptide). Fig. 5 shows the construction scheme in Reference Example 2 (1) (viii) for the plasmid pTF1. Fig. 6 shows the construction in Example 1 scheme for the plasmid pIFL 9906 ( [＿＿＿] indicates the portion coding

for human IFN-$\gamma$ peptide and ⧅⧅⧅⧅ indicates the portion coding for human IL-2 peptide).  Fig. 7 shows the result of measurement of NK activity in Example 3(2).

Reference Example 1:

Construction of plasmid pHITtrp 1101:

(1)  Construction of plasmid ptrp 771:

Plasmid ptrp 601 (the vector for which is pBR 322) containing the promoter portion for tryptophan synthesis in Escherichia coli [promoter- and operator-containing DNA segment with 276 base pairs, G.N.Bennett et al., J. Mol. Biol. 121, 113 (1978)] was constructed as the expression plasmid (Refer to British Patent Provisional Publication No. 2,102,006).

In a separate procedure, plasmid pBR 322 was digested with the restriction enzyme EcoRI and AvaI.  The recessed 3' ends of sticky ends of the resulting tetracycline-resistance gene-containing EcoRI-AvaI fragment filled with DNA polymerase I large fragment.  This fragment was ligated to the PvuII cleavage site of ptrp 601 using T4 DNA ligase, giving the plasmid ptrp 701 (Fig. 3).

Next, to remove one of the two cleavage sites of restriction enzyme ClaI present in ptrp 701, the plasmid was partially digested with ClaI.  This gave a ptrp 701 plasmid in which only one of the two ClaI cleavage sites has been cleaved.  After filling the recessed 3' ends with DNA polymerase I large fragment, the ptrp 701 was ligated with T4 DNA ligase to give ptrp 771 (Fig. 3).

(2)    Construction of plasmid pHITtrp 1101

The plasmid pHIT 3709 obtained by the method described in Japanese Patent Provisional Publication 58-189197(EPC Provisional Publication No.89676)  was cleaved with the restriction enzyme PstI to give a PstI fragment containing the structural gene for human IFN-γ .  This fragment was further partially digested with the restriction enzyme BstNI giving a BstNI-PstI fragment cleaved at the BstNI site present in the structural gene for human IFN-γ . The sticky ends at the BstNI cleavage site were filled with DNA polymerase I large fragment.  The fragment thus obtained was ligated with the oligonucleotide adapter

5'-CGATAATGTGTTACTGCC-3'
   TATTACACAATGACGG

which was chemically synthesized by the above mentioned triester method and contains the protein synthesis initiation codon ATG, using.T4 DNA ligase.

In a separate procedure, the human IFN-γ gene ligated with the above adapter was inserted, using T4 DNA ligase into the PstI-ClaI site of plasmid ptrp 771 downstream from the tryptophan promoter, giving pHIT trp 1101 plasmid that expresses human IFN-γ (Fig. 4).

(3) The E. coli strain 294/pHITtrp2101 was obtained by using the plasmid pHITtrp1101 obtained above in (2) to build the plasmid pHITtrp2101[J. Patent Provisional Publication 59-186995.EPC Provisional Publication No.110044)].

Reference Example 2

Construction of plasmid pTF1:

(1)(i)  Isolation of mRNA coding for human IL-2:

Lymphocytes prepared from the human peripheral blood were incubated in RPMI 1640 medium containing 10% fetal bovine serum supplemented with 12-o-tetradecanoylphorbol-13-acetate (TPA) (15 ng/ml) and concanavalin A (40 µg/ml) at 37 °C to induce IL-2.  After 24 hours, $1 \times 10^{10}$ human lymphocytes thus induced were disrupted and denatured in a solution containing 5M guanidine thiocyanate, 5% mercaptoethanol, 50 mM Tris-HCl (pH7.6), and 10mM EDTA with a Teflon homogenizer.  Next, sodium N-lauroyl sarcosinate was added to a concentration of 4%, and the mixture homogenized, then was layered onto 6 ml of 5.7 M cesium chloride solution (5.7 M cesium chloride, 0.1M  EDTA) and centrifuged using a Beckman SW28 rotor (Beckmann, U.S.A.) at 24,000 rpm and 15 °C for 48 hours to give an RNA precipitate.  This RNA precipitate was dissolved in 0.25% sodium N-lauroyl-sarcosinate and precipitated with ethanol to give 10 mg of RNA.  This RNA was adsorbed on an oligo(dT) cellulose column in a high-concentration salt solution (0.5M NaCl, 10 mM Tris-HCl pH 7.6, 1mM EDTA, 0.3% SDS).  Elution with a low-concentration salt solution (10 mM Tris-HCl pH 7.6, 1 mM EDTA, 0.3% SDS) gave 300 µg of poly(A)-containing mRNA.  This mRNA was again precipitated with ethanol, then dissolved in 0.2 ml of a solution (10 mM Tris-HCl pH 7.6, 2 mM EDTA, 0.3% SDS), treated at 65 °C for 2 minutes and fractionated by 10-35% sucrose density gradient centrifugation (at 20 °C and 25,000 rpm for 21 hours using a Beckmann SW28 rotor) into 22 fractions. An aliquot of each fraction was injected into oocytes of <u>Xenopus</u> <u>laevis</u> and the IL-2 activity in proteins thus synthesized was measured. Fraction Nos. 11-15 (sedimentation coefficient 8S-15S) were found to have IL-2 activity. About 25 µg of IL-2 mRNA was contained in these fractions.

(ii)  Synthesis of single-stranded DNA:

Using the mRNA obtained above and reverse transcriptase, incubation was carried out in 100 µl of reaction solution (5 µg of mRNA, 50 µg of oligo(dT), 100 units of reverse transcriptase, 1 mM dATP, 1 mM dCTP, 1 mM dGTP, 1 mM TTP, 8 mM MgCl$_2$, 50 mM KCl, 10 mM dithiothreitol, 50 mM Tris-HCl pH 8.3) at 42°C for 1 hour, followed by deproteinization with phenol and treatment with 0.1 N NaOH at 70°C for 20 minutes for removal of RNA by decomposition.

(iii)  Synthesis of double-stranded DNA:

A double-stranded DNA was synthesized by treating the single-stranded complementary DNA thus synthesized in 50 µl of a reaction solution [identical to that above mentioned except for the absence of mRNA and oligo(dT)] at 42°C for 2 hours.

(iv)  Addition of dC tail:

This double-stranded DNA was treated with S1 nuclease in 50 µl of a reaction solution (double-stranded DNA, 0.1 M sodium acetate, pH 4.5, 0.25 M NaCl, 1.5mM ZnSO$_4$, and 60 units of S1 nuclease) at room temperature for 30 minutes, followed by deproteinization with phenol and DNA precipitation with ethanol. The DNA was treated with terminal transferase in 50 $\mu$l of a reaction medium (double-stranded DNA, 0.14 M potassium cacodylate, 0.3 M Tris (base) pH 7.6, 2 mM dithiothreitol, 1 mM $CoCl_2$, 0.15 mM dCTP, 30 units of terminal transferase) at 37°C for 3 minutes to extend the double-stranded DNA by about deoxycytidinechains of about 15 nucleotides at both 3' terminals.  This series of reactions gave about 300 ng of poly-deoxycytidine tailed doublestranded DNA.

(v)  Cleavage of Escherichia coli plasmid and addition of dG tail

In a separate procedure, 10 $\mu$g of Escherichia coli plasmid pBR 322 DNA was treated with the restriction enzyme PstI in 50 $\mu$l of a reaction medium (10 $\mu$g of DNA, 50 mM NaCl, 6mM Tris-HCl, pH 7.4, 6 mM $MgCl_2$, 6 mM 2-mercaptoethanol, 100 $\mu$g/ml bovine serum albumin, 20 units of PstI) at 37°C for 3 hours to cleave the one PstI recognition site in the pBR 322 DNA, then deproteinized with phenol.  The cleaved plasmid pBR 322 DNA was extended by a deoxyguanine chain of about 17 nucleotides at both 3' terminals by treating with terminal transferase in 50 $\mu$l of a reaction solution (10 $\mu$g of DNA, 0.14 M potassium cacodylate, 0.3 M Tris base pH 7.6, 2 mM dithiothreitol, 1 mM $CoCl_2$, 0.15 mM GTP, 30 units of terminal deoxynucleotidyl transferase) at 37°C for 3 minutes.

(vi)  Annealing of cDNA with pBR322 DNA and transformation of Escherichia coli:

The synthetic double-stranded DNA thus obtained (0.1

μg) and 0.5 μg of the above mentioned plasmid pBR 322 were annealed by heating in a solution containing 0.1 M NaCl, 50 mM Tris-HCl pH 7.6 and 1 mM EDTA at 65 °C for 2 minutes and then at 45 °C for 2 hours followed by gradual cooling and the product used to transform Escherichia coli MM294 in accordance with the method of Enea et al. [J. Mol. Biol. 96, 495 (1975)]

(vii) Isolation of cDNA-containing plasmid:

In this way, about 20,000 tetracycline-resistant transformants were isolated. The DNAs of each of these were fixed on a nitrocellulose filter. Based on the amino acid sequence of IL-2 reported by Taniguchi et al.[Nature 302, 305 (1983)], the complementary oligonucleotides of the base sequences ($^{5'}$AAA CAT CTT CAG TGT$^{3'}$ and $^{5'}$ACA TTC ATG TGT GAA$^{3'}$) corresponding to amino acids 74-78(Lys$^{74}$-His-Leu-Gln-Cys) and amino acids 122-126(Thr$^{122}$-Phe-Met-Cys-Glu), were chemically synthesized by the phosphotriester method [Crsa, R. et al., Proc. Natl. Acad. Sci. USA 75, 5765 (1978)]. These oligonucleotides were labeled with $^{32}$P at the 5' terminal by treatment with T4 polynucleotide kinase in 50 μl of a reaction solution ) 0.20 μg of oligonucleotide, 50 mM Tris-HCl, pH 8.0, 10 mM MgCl$_2$, 10 mM 2-mercaptoethanol, 50 μCi of γ-$^{32}$P ATP, 3 units of T4 polynucleotide kinase) at 37 °C for 1 hour. These labeled oligonucleotides were hybridized as probes with the above-mentioned DNAs fixed on nitrocellulose filter by the method of Lawn et al. [Nucleic Acids Res. 9, 6103 (1981)]. Four transformants reactive with the above two oligonucleotide probes were isolated by autoradiography.  The plasmid DNA was isolated from each of these transformants by the Birnboim-Doly alkali method [Birnboim, H.C. & Doly, J., Nucleic Acids Res., 7, 1513, (1979)].  Then the insert part in the plasmid DNA was removed using the restriction enzyme PstI.  Of the plasmids isolated, that containing the longest insert fragment was selected and named pILOT 135-8.

(viii)  Construction of plasmid pTF1:

A DNA fragment having 1294 base pairs was obtained by digesting the plasmid pILOT 135-8 obtained in (vii) above with the restriction enzyme HgiAI.  The fragment was treated with T4 DNA polymerase to form flush ends and the EcoRI linker p(TGCCATGAATTCATGGCA) ligated with T4 DNA ligase.  The ligation reaction was followed by the cleavage by EcoRI to make each end of the fragment linked by single EcoRI linker.  Then the fragment was cleaved with the restriction enzyme PstI.  As the result of these process, the initiation codon ATG was then adapted as the initiation of the reading frame of the human IL-2 gene without its portion coding the signal peptide.

The resulting DNA fragment was treated with T4 DNA ligase, to join the expression plasmid ptrp 781 cleaved with restriction enzyme EcoRI and PstI [Nucleic Acids Research 11, 3077-3085 (1983)].

The above reaction, gave a human IL-2 expression plasmid pTF1 having initiation codon downstream from trp promoter, as the initiation of its reading frame was obtained (Fig 5). A strain carrying the plasmid pTF1 was obtained by transforming *Escherichia coli* DH1 with the plasmid.

(2) The *Escherichia coli* DHI/pTF4 obtained by using the plasmid pILOT 135-8 obtained above in (1) to build the plasmid pTF4, and using this to transform *E. coli* DHI, was deposited at the IFO under ascession number IFO 14299 on November 25, 1983. This organism was also deposited at the FRI as FERM P-7578 on April 6, 1984. This deposit was converted to a deposit under the Budapest Treaty, and stored at the FRI as FERM BP-628.

The transformant was prepared as follows. Plasmid pILOT 35-8 obtained in (1) above was cleaved with restriction enzyme HgiAI, giving a DNA segment containing the IL-2 gene of 1249bp. This segment was treated with T4 DNA polymerase, then the Cla I linker

<div align="center">

5'-CGATAATGGCA-3'
TATTACCGT

</div>

which bears an alanine codon GCA and a methionine codon AGT, was attached, and linker-bearing DNA segment treated with Cla I and Pst I. Next, this segment was inserted at the Cla I and Pst I sites of the ptrp 771 using T4 DNA ligase, and the expression plasmid obtained named pTF4. This plasmid pTF4 was used to transform *E. coli* DHI by the method described by Cohen and his colleagues [Proc. Natl. Acad. Sci. USA, 69, 2110 (1972)], and a transformant (*E. coli* DHI/pTF4) containing this plasmid was obtained.

(3) Production of the human IL-2 protein:

E. coli DHI/pTF4 obtained above in (2) was inoculated into 50 ml of a liquid medium (pH 7.0) containing 1% Bacto tryptone (Difco Laboratories, USA), 0.5% Bacto yeast extract (Difco Laboratories, USA), 0.5% sodium chloride and 7 μg/ml tetracycline as placed in a 250-ml erlenmeyer flask. After incubation at 37°C overnight on a swing rotor, the culture medium was transferred to a 5-liter jar fermenter containing 2.5 liters of M9 medium containing 0.5% casamino acids, 0.5% glucose and 7 μg/ml tetracycline. Incubation was then conducted with aeration and stirring at 37°C for 4 hours and, after addition of 3-β-indolylacrylic acid (25 μg/ml), for further 4 hours. Cells were harvested from the thus-obtained 2.5-liter culture broth by centrifugation, frozen at -80°C and stored.

The freeze-stored cells (12.1 g) obtained above were suspended in 100 ml of an extractant (pH 7.0) containing 7 M guanidine hydrochloride and 0.1 M Tris HCl, the suspension was stirred at 4°C for 1 hour and the lysate was centrifuged at 28,000 x g for 20 minutes to obtain 93 ml of a supernatant.

The supernatant fluid obtained above was dialyzed against 0.01 M Tris-HCl buffer (pH 8.5) and then centrifuged at 19,000 x g for 10 minutes to give 94 ml of a dialyzed supernatant fluid. This was applied to a DE 52 (DEAE-cellulose, Whatman, Great Britain) column (50 ml in volume) equilibrated with 0.01 M Tris-HCl buffer (pH 8.5) for protein adsorption. Proteins were eluted with a linear NaCl concentration gradient (0-0.15 M NaCl, 1 liter). The active fractions (53 ml) were concentrated to 4.8 ml using a YM-5 membrane (Amicon, USA) and subjected to gel filtraction using a Sephacryl S-200 (Pharmacia, Sweden) column (500 ml in volume) equibrated with 0.1 M Tris HCl (pH 8.0)-1 M NaCl buffer. The active fractions (28 ml) obtained were concentrated to 2.5 ml using a YM-5 membrane. The concentrate was applied to an Ultrapore RPSC (Altex, USA) column for adsoprtion, and high performance liquid chromatography was performed using a trifluoroacetic acid – acetonitrile system as the mobile phase.

The conditions used:  column, Ultrapore RPSC (4.6 x 75 mm); column temperature, 30 °C; solvent A, 0.1% trifluoroacetic acid-99.9% water; solvent B, 0.1% trifluoroacetic acid-99.9% acetonitrile; elution program, minute 0 (68% A + 32% B) – minute 25 (55% A + 45% B) – minute 35 (45% A + 55% B) – minute 45 (30% A + 70% B) – minute 48 (100% B); elution rate, 0.8 ml/min.;  detection wave length, 230 nm. An active fraction was collected at a retention time of about 39 minutes. Thus was obtained 10 ml of a solution containing 0.53 mg of non-glycosylated human IL-2 protein [specific activity, 30,000 U/mg; activity recovery from the starting material, 30.6% purity of protein, 99% (determined by densitometry on an SDS-polyacrylamide gel electrophorefogram)].

Lyophilization of the above solution gave a white powder.  The powder had a specific activity of 26,000 U/mg.

The human IL-2 protein thus obtained is a peptide for which above-mentioned DNA represented by (V) codes.

Example 1

Construction of plasmid pIFL9906 expressing the polypeptide of ILR-$\gamma$ 2 and production of the transformant carrying same:

A DNA fragment of about 450 base pairs long was obtained by cleaving the human IFN-$\gamma$ expression plasmid pHITtrp 1101 obtained in Reference Example 1 with the restriction enzymes HinfI and HpaI.  The DNA fragment was treated with Escherichia coli DNA polymerase I large fragment to form the flush ends.  EcoRI linker-p(CCGGAATTCCGG) was ligated to this fragment using T4 DNA ligase and digested with restriction enzymes EcoRI and ClaI to form sticky ends.

In a separate procedure, a DNA fragment of about 450 base pairs long was obtained by cleaving the human IL-2 expression plasmid pTF1 obtained in Reference Example 2 with the restriction enzymes ClaI and PstI.  These two DNA fragments were ligated with T4 DNA ligase and the plasmid pIFL 9906 constructed by integrating the resulting DNA fragment to ptrp 771 [See Reference Example 1 (1), Japanese patent provisional publication  No.59-44399, and EPC Provisional Publication No. 102, 634] cleaved with the restriction enzyme ClaI and PstI (See Fig. 6).

The strain E.coli DH1/pIFL9906 (IFO 14331, FERM BP-711) carrying the plasmid pIFL 9906 was obtained by transforming Escherichia coli DH1 with the plasmid pIFL 9906.

Example 2

(1) Preparation of supernatant of organisms containing ILR-$\gamma$ 2:

The transformant E. coli DH1/pIFL9906 (IFO 14331, FERM BP-711) carrying the plasmid pIFL 9906 for ILR-$\gamma$ 2 expression was cultivated in 20 ml of M9 medium containing 1% glucose and 0.4% Casamino acids at 37 °C for 4 hours and cultivated at 37 °C for another 3 hours following the addition of indolylacrylic acid (30 μg/ml). The cells were collected and washed with NaCl solution. These were then suspended in 0.5 ml of an extractant (10 ml Tris-HCl, pH 8.0, 10 ml EDTA, 0.2 M NaCl, 1 mM phenylmethylsulphonyl fluoride, 0.02% Triton X100, 0.1 mg/ml lysozyme) and allowed to stand at 0 °C for 45 minutes, then incubated at 37 °C for 2 minutes to lyse the cells. The thus obtained cell lysate was ultrasonically treated for 30 seconds and the supernatant was obtained by centrifugation at 15,000 rpm (Serval SS34 rotor, Du pondte, U.S.A.) for 30 minutes at 4 °C as an extract of the cells.

(2) Measurement of IFN-$\gamma$ activity in the extract:

The antiviral activity of the extract obtained in (1) was measured by running a test that evaluates the cell degenaration inhibiting effect on the vesicular stomatitis virus (VSV) in human amnion-derived WISH cells. The IFN-$\gamma$ activity was 2 X $10^6$ units per liter of broth.

(3) Measurement of IL-2 in the extract

The activity of human IL-2 was measured by using a TCGF-dependent mouse cell line (NKC3) [Conference of Japan

Immune Society $\underline{11}$,'277(1981)]. First, a 50 $\mu$l aliquotes of IL-2 containing sample diluted to various concentrations by two-step dilution were placed in flat-bottomed microtiter plates (Falcon, U.S.A.). Then 50 $\mu$l of RPMI-1640 medium containing 10% fetal bovine serum (10% FCS) and 3 $\times 10^4$ of NKC3 cells were added to the plate and incubated at 37°C for 20 hours in a $CO_2$ incubator. Tritiated thymidine (1 $\mu$Ci) was added to each plate and after continued incubation for 4 hours, the cells were recovered onto a glass filter using a cell harvester (Wakenyaku Kogyo Japan). After washing, filtration, and drying, the radioactivity of tritiated thymidine taken up was measured with a liquid scintillation counter. The IL-2 activity of ILR-$\gamma$ 2 in the sample solution was 13 $\times 10^3$ units per liter of broth.

The activity mentioned above was measured based on the method of calculation described in Japanese Patent Provisional Publication 58-116498 (EPC Provisional Publication No. 88195).

(4)  Purification of ILR-$\gamma$ 2:

The anti-human IFN-$\gamma$ polyclonal antibody was coupled with the water insoluble carrier AFFI-GEL 10 (Bio-Rad Lab, U.S.A.), using the method described in Japanese Patent Application 58-176090 [Japanese Provisional Publication No. 59-186995 (EPC Provisional Publication No.110044)], 58-176091 (Japanese Provisional Publication No. 59-80646, EPC Provisional Publication No. 103, 898). Five ml of the extract containing $\underline{E.coli}$ DH1/pIFL 9906 obtained in (3) was passed through an anti-human IFN-$\gamma$ polyclonal antibody-AFFI-GEL 10 column. After washing the column with 50 ml of PBS (20 mM phosphate buffer, pH6.8, 0.15 M NaCl) containing 20% dextrose, ILR-$\gamma$ 2 was eluted with 5 ml of 0.2 M acetic acid containing 0.15 M NaCl. The eluate was immediately neutralized and the resulting solution was dialyzed at 5 °C for 24 hours against one liter of a PBS solution. This treatment gave an ILR-$\gamma$ 2 polypeptide of over 80% purity, with approximately 50%.

Example 3

(1) Preparation of the supernatant of cells containing ILR-$\gamma$ 2:

The transformant E. coli DHl/pIFL 9906 (IFO 14331, FERM BP-711) containing the plasmid pIFL 9906 for ILR-$\gamma$ 2 expression was incubated in 200 ml of M9 medium containing 1% glucose and 0.4% Casamino acid at 37 °C for 4 hours. This was followed by the addition of indolylacrylic acid (30 $\mu$g/ml), incubation continued for 3 hours at 37 °C. After collection and washing with saline solution, the cells were suspended in 20 ml of an extractant (0.1 M Tris-HCl pH 8, 7M guanidine-HCl) and lysed by being allowed to stand at 0 °C for 1.5 hours. The supernatant was obtained by two 30-minute centrifugations at 15,000 rpm (Serval SS34 rotor) at 4 °C.

The supernatant thus obtained was dialysed against 10 liters of Tris-HCl solution (10 mM, pH 8.5) and 10mM of glysine-Na (pH 6.5) at 4 °C for 3 hours, then centrifuged at 15,000rpm (Servel SS34 rotor) for 20 minutes at 4 °C to give another supernatant. This was submitted to CM-Sephadex (C-50) column chromatography (pH 6.5), eluted with 10 mM glysine-Na (pH6.5) and 0.4-1.0 M NaCl as the extract, and the fractions showing some activity collected.

(2) Measurement of natural killer (NK) activity in ILR-$\gamma$ 2:

The activity of natural killer was measured in accordance with the method by R.B.Herberman and H.T.Holden (Adv. Cancer Res. $\underline{21}$, 305 (1978)). Human K 562 cells (erythroleukemia cell) were incubated in a complete minimal essential medium (CMEM) as target cells and $4\text{-}6\text{X}10^6$ cells labeled by $Na^{51}CrO_4$. Human peripheral blood mononuclea cells (PBMC) were used as the effector cells. PBMC ($3 \times 10^6$/ml) -has been activated by incubating in a CMEM. An amount of 0.1 ml of the labeled target cells ($1 \times 10^5$/ml) was added to 0.1 ml of the effector cells. The ratio of effector cells to target cells was varied as follows: 2.5:1, 5:1, 10:1. After allowing these to stand at 37°C overnight, the supernatant of the incubation broth was collected and was measured with a gamma-counter. $^{51}Cr$ naturally released when target cells alone were left to stand in CMEM was shown only 15%. The percentage of NK activity was calculated as follows:

$$\left( \frac{\text{experimental value of } ^{51}Cr \text{ released} - \text{value of } ^{51}Cr \text{ naturally released}}{\text{maximum value of } ^{51}Cr \text{ released} - \text{value of } ^{51}Cr \text{ naturally released}} \right) \times 100$$

(all values in cpm)

The values thus measured were studied in the presence of no more than 0.1 unit of IL-2, 10 units of IFN-$\gamma$ , ILR-$\gamma$ 2 (equivalent to 0.1 unit of IL-2 and 10 units of IFN-$\gamma$ ).

The peptide obtained above in Reference Example 2 was used as IL-2. And the peptide obtained by a method described in Japanese Patent Provisional Publication No. 58-189197(EPC Provisional Publication No.89676) was used as IFN-$\gamma$ . The results revealed, as shown in Fig. 7, that the NK activity of ILR-$\gamma$ 2 was stronger than that of IL-2 or IFN-$\gamma$ alone.

In Fig. 7, $-\blacktriangle -$ shows a plot of IFN-$\gamma$ (10 U), $-\blacksquare -$ shows a plot of IL-2 (0.1 U), $-$ o $-$ shows a plot of ILR-$\gamma$ 2 (IL-2, 0.1 U: IFN-$\gamma$ , 10 U), respectively.

By using ILR-$\gamma$ 2 according to the present invention, human IL-2 can be refined effectively. And ILR-$\gamma$ 2 according to the present invention may be used as an anti-tumor agent.

CLAIMS

1. DNA made by ligating a DNA containing a structural gene coding for a peptide of human immune interferon and a DNA containing a structural gene coding for a peptide of human interleukine-2 to code the peptide of human IFN-$\gamma$ and the peptide of human IL-2 in a single reading frame.

2. A plasmid carrying a DNA made by ligating a DNA containing a structural gene coding for a peptide of human immune interferon and a DNA containing a structural gene coding for a peptide of human interleukine-2 to code the peptide of human IFN-$\gamma$ and the peptide of human IL-2 in a single reading frame.

3. A transformant obtained with a plasmid carrying a DNA made by ligating a DNA containing a structural gene coding for a peptide of human immune interferon and a DNA containing a structural gene coding for a peptide of human interleukine-2 to code the peptide of human IFN-$\gamma$ and the peptide of human IL-2 in a single reading frame.

4. A transformant according to claim 3, wherein the host of the transformant belongs to the genus Escherichia.

5. A polypeptide consisting of a peptide of human immune interferon and a peptide of human interleukine-2.

6. A method of producing polypeptide comprising a peptide of human immune interferon and a peptide of human interleukine-2, which comprises cultivating a transformant obtained with a plasmid carrying a DNA made by ligating a DNA containing a structural gene coding for a peptide of human interferon and a DNA containing a structural gene coding for a peptide of human interleukine-2 to code the peptide of human IFN-γ and the peptide of human IL-2 in a single reading frame in a culture medium, having the transformant produce and accumulate said polypeptide in the culture broth, and isolating the polypeptide from the broth.

7. A method according to claim 6, wherein the host of the transformant belongs to the genus Escherichia.

8. A method of producing a DNA according to claim 1 comprising ligating a DNA containing a structural gene coding for a peptide of human immune interferon and a DNA containing a structural gene coding for a peptide of human interleukin-2 to code the peptide of human IFN-γ and the peptide of human IL-2 in a single reading frame.

9. A method of producing a plasmid according to claim 2 comprising integrating a DNA made by ligating a DNA

0158198

containing a structural gene coding for a peptide of human immune interferon and a DNA containing a structural gene coding for a peptide of human interleukin-2 to code the peptide of human IFN-γ and the peptide of human IL-2 in a single reading frame, into a plasmid DNA.

10. A method of obtaining a transformant according to claim 3 comprising introducing a plasmid having integrated therein a DNA made by ligating a DNA containing a structural gene coding for a peptide of human immune interferon and a DNA containing a structural gene coding for a peptide of human interleukin-2 to code the peptide of human IFN-γ and the peptide of human IL-2 in a single reading frame, into a host.

41

## Figure 1

```
        10        20        30        40        50
         *         *         *         *         *
ATCGATGTGTTACTGCCAGGACCCATATGTAAAAGAAGCAGAAAACCTTA      50
AGAAATATTTTAATGCAGGTCATTCAGATGTAGCGGATAATGGAACTCTT     100
TTCTTAGGCATTTTGAAGAATTGGAAAGAGGAGAGTGACAGAAAAATAAT     150
GCAGAGCCAAATTGTCTCCTTTTACTTCAAACTTTTTAAAAACTTTAAAG     200
ATGACCAGAGCATCCAAAAGAGTGTGGAGACCATCAAGGAAGACATGAAT     250
GTCAAGTTTTTCAATAGCAACAAAAAGAAACGAGATGACTTCGAAAAGCT     300
GACTAATTATTCGGTAACTGACTTGAATGTCCAACGCAAAGCAATACATG     350
AACTCATCCAAGTGATGGCTGAACTGTCGCCAGCAGCTAAAACAGGGAAG     400
CGAAAAAGGAGTCCGGAATTCATGGCACCTACTTCAAGTTCTACAAAGAA     450
AACACAGCTACAACTGGAGCATTTACTGCTGGATTTACAGATGATTTTGA     500
ATGGAATTAATAATTACAAGAATCCCAAACTCACCAGGATGCTCACATTT     550
AAGTTTTACATGCCCAAGAAGGCCACAGAACTGAAACATCTTCAGTGTCT     600
AGAAGAAGAACTCAAACCTCTGGAGGAAGTGCTAAATTTAGCTCAAAGCA     650
AAAACTTTCACTTAAGACCCAGGGACTTAATCAGCAATATCAACGTAATA     700
GTTCTGGAACTAAAGGGATCTGAAACAACATTCATGTGTGAATATGCTGA     750
TGAGACAGCAACCATTGTAGAATTTCTGAACAGATGGATTACCTTTTGTC     800
AAAGCATCATCTCAACACTAACTTGATAATTAAGTGCTTCCCACTTAAAA     850
CATATCAGGCCTTCTATTTATTTAAATATTTAAATTTTACCCCCCCCCCC     900
CCCC
```

TOTAL NUMBER OF NUCLEOTIDE PAIRS =        904

2/4

Figure 2

MET CYS TYR CYS GLN ASP PRO TYR VAL LYS GLU ALA GLU ASN LEU

LYS LYS TYR PHE ASN ALA GLY HIS SER ASP VAL ALA ASP ASN GLY

THR LEU PHE LEU GLY ILE LEU LYS ASN TRP LYS GLU GLU SER ASP

ARG LYS ILE MET GLN SER GLN ILE VAL SER PHE TYR PHE LYS LEU

PHE LYS ASN PHE LYS ASP ASP GLN SER ILE GLN LYS SER VAL GLU

THR ILE LYS GLU ASP MET ASN VAL LYS PHE PHE ASN SER ASN LYS

LYS LYS ARG ASP ASP PHE GLU LYS LEU THR ASN TYR SER VAL THR

ASP LEU ASN VAL GLN ARG LYS ALA ILE HIS GLU LEU ILE GLN VAL

MET ALA GLU LEU SER PRO ALA ALA LYS THR GLY LYS ARG LYS ARG

SER PRO GLU PHE MET ALA PRO THR SER SER SER THR LYS LYS THR

GLN LEU GLN LEU GLU HIS LEU LEU LEU ASP LEU GLN MET ILE LEU

ASN GLY ILE ASN ASN TYR LYS ASN PRO LYS LEU THR ARG MET LEU

THR PHE LYS PHE TYR MET PRO LYS LYS ALA THR GLU LEU LYS HIS

LEU GLN CYS LEU GLU GLU GLU LEU LYS PRO LEU GLU GLU VAL LEU

ASN LEU ALA GLN SER LYS ASN PHE HIS LEU ARG PRO ARG ASP LEU

ILE SER ASN ILE ASN VAL ILE VAL LEU GLU LEU LYS GLY SER GLU

THR THR PHE MET CYS GLU TYR ALA ASP GLU THR ALA THR ILE VAL

GLU PHE LEU ASN ARG TRP ILE THR PHE CYS GLN SER ILE ILE SER

THR LEU THR

Figure 3

Figure 4

Figure 5

MetAlaProThr --------
AATTCATGGCACCTACT ----------- CTGCA
GTACCGTGGATGA ----------- G

Figure 6

Figure 7

## European Patent Office

# EUROPEAN SEARCH REPORT

| | DOCUMENTS CONSIDERED TO BE RELEVANT | | EP 85103450.4 |
|---|---|---|---|

| Category | Citation of document with indication. where appropriate, of relevant passages | Relevant to claim | CLASSIFICATION OF THE APPLICATION (Int Cl 4) |
|---|---|---|---|
| D,P, A | EP - A1 - 0 126 230 (TAKEDA CHEMI-CAL INDUSTRIES, LTD.) <br> * Claims 1,6,9,11-14 * | 1-10 | C 12 N 15/00 <br> C 12 P 19/34 <br> C 12 P 21/02 <br> C 07 K 7/10 |
| D,A | EP - A2 - 0 095 350 (SUNTORY KABUSHIKI KAISHA) <br> * Claims 1-6,10,13,16 * | 1-10 | C 07 K 15/26 <br> //C 12 R 1:185 |
| D,A | EP - A2 - 0 089 676 (TAKEDA CHEMI-CAL INDUSTRIES, LTD.) <br> * Claims 1,6,18 * | 1,6 | |
| D,A | EP - A2 - 0 088 540 (BIOGEN N.V.) <br> * Claims 1,19,20,22,23,25 * | 1,5-8 | |
| A | EP - A1 - 0 091 539 (AJINOMOTO, JAPANESE FOUNDATION FOR CANCER RESEARCH) <br> * Claims 19,21,29; fig. 2 * | 1,5-7 | TECHNICAL FIELDS SEARCHED (Int Cl 4) <br><br> C 12 N <br> C 12 P <br> C 07 K |
| D,A | NATURE, vol. 302, March 24, 1983 (New York, London) <br><br> T. TANIGUCHI et al. "Structure and expression of a cloned cDNA for human interleukin-2" pages 305-310 <br><br> * Totality * | 1 | |

The present search report has been drawn up for all claims

| Place of search <br> VIENNA | Date of completion of the search <br> 03-07-1985 | Examiner <br> WOLF |
|---|---|---|

# DECLARATION PURSUANT TO RULE 28, PARAGRAPH 4, OF THE EUROPEAN PATENT CONVENTION

The applicant has informed the European Patent Office that, until the publication of the mention of the grant of the European patent or until the date on which the application has been refused or withdrawn or is deemed to be withdrawn, the availability of the micro-organism(s) identified below, referred to in paragraph 3 of Rule 28 of the European Patent Convention, shall be effected only by the issue of a sample to an expert.

## IDENTIFICATION OF THE MICRO-ORGANISMS

Accession numbers of the deposits:     IFO  14331

FERM  BP-711

FERM  BP-628